# EUROPEAN PATENT APPLICATION

(11) **EP 1 398 632 A1**
(43) Date of publication of application: **17.03.2004**
(21) Application number: 02020415.2
(22) Date of filing: 11.09.2002
(51) Int. Cl.: G01N 33/50, C07K 14/72

(54) **P2y11 receptor agonists and the use in the field of erythropoiesis**

(71) Applicant: Bayer HealthCare AG, 51368 Leverkusen (DE)
(72) Inventor: Kauschat, Dörte Dr., 42113 Wuppertal (DE); Fröhlen, Britta Dr., 51377 Leverkusen (DE)

(57) **Abstract**

The invention relates to novel disease associations of P2Y₁₁R and / or P2Y₁₁R agonists for the prophylaxis, treatment and / or diagnosis of anemia, in particular the prophylaxis, treatment and / or diagnosis of pure red cell aplasia, anemia of chronic renal failure, anemia of endocrine disorders, congenital dyserythropoietic anemia, iron deficiency, congenital atransferrinemia and idiopathic pulmonary hemosiderosis. Furthermore, methods of screening for novel disease association P2Y₁₁R agonists are provided.

## Description

### Technical Field of the Invention

The present invention is in the field of erythropoiesis, in particular, the present invention describes the use of P2Y₁₁R agonists for the prophylaxis, and / or treatment of anemia.

### Background of invention

### Anemia

Anemia is a disorder in which the patient suffers from tissue hypoxia, the consequence of a low oxygen-carrying capacity of the blood. On the basis of determination of red cell mass, anemia can be classified as relative anemia (macro-globulinemia, pregnancy, nutritional deficiency and splenomegaly) and absolute anemia predominantly caused by decreased red cell production or caused predominately by increased erythrocyte destruction or loss.

Aplastic anemia and anemia of leukemia and of myelodysplastic syndromes are caused by disturbance of proliferation and differentiation of hematopoietic stem cells. The disturbance of proliferation and differentiation of erythroid progenitors and precursor cells results in pure red cell aplasia, anemia of chronic renal failure, anemia of endocrine disorders and congenital dyserythropoietic anemia. Iron deficiency, congenital atransferrinemia and idiopathic pulmonary hemosiderosis are caused by disturbance of hemoglobin synthesis.

Hemoglobin in red blood cells is the key component for transporting oxygen from the lung to the tissues. In anemia the level of hemoglobin has fallen below 12 g/L (Hemoglobin in normal male population ranged between 14.0-17.5 g/dl, and in normal female population from 12.3-15.3 g/dl). Common reasons for anemia include acute or chronic blood loss, insufficient levels of erythropoietin synthesis in the kidneys (e.g. in dialysis patients) or insufficient output of red blood cells from bone marrow after chemotherapy or HIV infection etc. Current therapy of anemia is aimed at increasing the hematocrit either by transfusion or by stimulating erythropoiesis with agents such as erythropoietin. The intention of the treatment is to restore hemoglobin levels above 12 g/L.

### Receptors for Purines and Pyrimidines

Extracellular purines (Adenosine, ADP and ATP) and pyrimidines (UDP and UTP) are important signaling molecules that mediate diverse biological effects via cell surface receptors termed purine receptors. There are two main families of purine receptors, adenosine or P1 receptors, and P2 receptors, recognizing primarily ATP, ADP, UTP, and UDP. Adenosine/P1 receptors have been divided into four subgroups, A₁, A_{2A}, A_{2B}, and A₃, coupling to G protein (Ralevic et. *al.* 1998).

Based on differences in molecular structure and signal transduction mechanisms, P2 receptors divide naturally into two families of ligand-gated ion channels (P2X ₁₋₇) and G protein-coupled receptors (P2Y_{1, 2, 4, 6, 11, 12, 13}), (Ralevic *et.* al 1998, Moore et.*al. 2001, Communi et. al 2001*).

P2Y receptor subtypes mediate the activation of phospholipase C (PLC) and formation of Inositolphosphate (IP3). PLC and IP3 mediate the activation of protein kinase C and the release of intracellular calcium (Ralevic *et. al* 1998).

P2Y₁₁ receptor (SEQ ID NO:1) stimulated by ATP is distinct from all other members of its family by the way that it is coupled to the adenylyl cyclase pathway as well as the IP3 pathway and mediate the enhancement of cyclic AMP synthesis (Qi *et. al* 2001).

As shown of other examples P2Y receptors agonists or antagonists have pharmacological properties. The involvement of P2Y₁ on platelet aggregation ( Savi *et. al* 1998), or P2Y₂, whose activation leads to an enhancement of chloride ions efflux and thus a potential target for the treatment of cystic fibrosis (O'Reilly *et. al* 1998), are examples of potential therapeutic significance of individually receptor subtypes.

### Brief Description of the Figures

### Figure 1

Relative expression of P2Y₁₁R, Glycophorin A (GPA), hemoglobinl/2 alpha (Hb1/2α), and hemoglobin beta (Hb β) mRNA transcripts were analyzed in primary bone marrow (BM) cells. Total RNA from bone marrow CD34⁺ (columns 1, 3, 5, 7) cells and CD71⁺ (columns 2, 4, 6, 8) cells were isolated. After cDNA synthesis TaqMan analyses for the expression of P2Y₁₁R (column 1, 2), GPA (column 3, 4), Hbl/2α (column 3, 4), and Hb β (column 7, 8) was performed. Relative expression of mRNA transcripts were calculated as described in paragraph expression profile. The y-axis shows the relative expression in arbitrary units.

### Figure 2

Relative expression of P2Y_{I1}R, granulocyte colony stimulating factor receptor (G-CSF-R), and CD11b mRNA transcript were analyzed in primary bone marrow cells in BM CD34⁺ (columns 1, 3, 5) cells and BM CD15⁺ (columns 2, 4, 6). Total RNA from BM CD34⁺ cells and BM CD15⁺ cells were isolated. After cDNA synthesis TaqMan Analysis for the expression of P2Y₁₁R (column 1, 2), G-CSF-R (column 3, 4), CD11b (column 5, 6) was performed. Relative expression of mRNA transcripts were calculated as described in paragraph expression profile. The y-axis shows the relative expression in arbitrary units.

### Detailed Description of the Invention

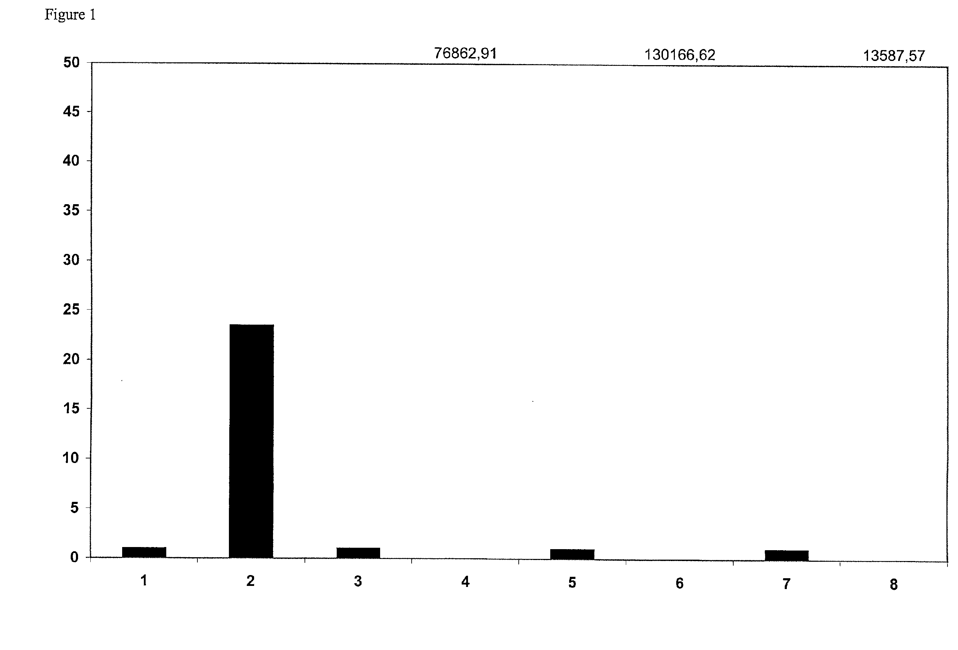

Gene expressing profiling of different hematopoietic stem cells revealed that in addition to the expression of P2Y₁₁R in promyelocytic cells lines (van der Weyden*et. al* 2000) P2Y₁₁R mRNA is expressed in primary erythroid cells. Unexpectedly, the quantitative analysis of P2Y₁₁R mRNA expression in human hematopoietic bone marrow cells showed higher expression in erythroid progenitor cells (CD71⁺ cells, Figure 1, column 2) than in early progenitor cells (CD34⁺ cells) (Figure 1, column 1). CD71⁺ cells purified from bone marrow have a definite erythroid character. Glycophorin A (GPA) as well as hemoglobin1/2 alpha and Hemoglobin beta are upregulated during the differentiation process. For the evaluation of the erythroid character of bone marrow cells used for the expression profile of P2Y₁₁R the regulation of hemoglobin and GPA was analyzed. Both hemoglobin (1/2 alpha and beta) (Figure 1, column 5 H1/2 α, column 8 Hb β) as well as the mRNA transcript for GPA (Figure 1, column 4) is clearly increased in CD71⁺ cells compared to CD34⁺ cells.

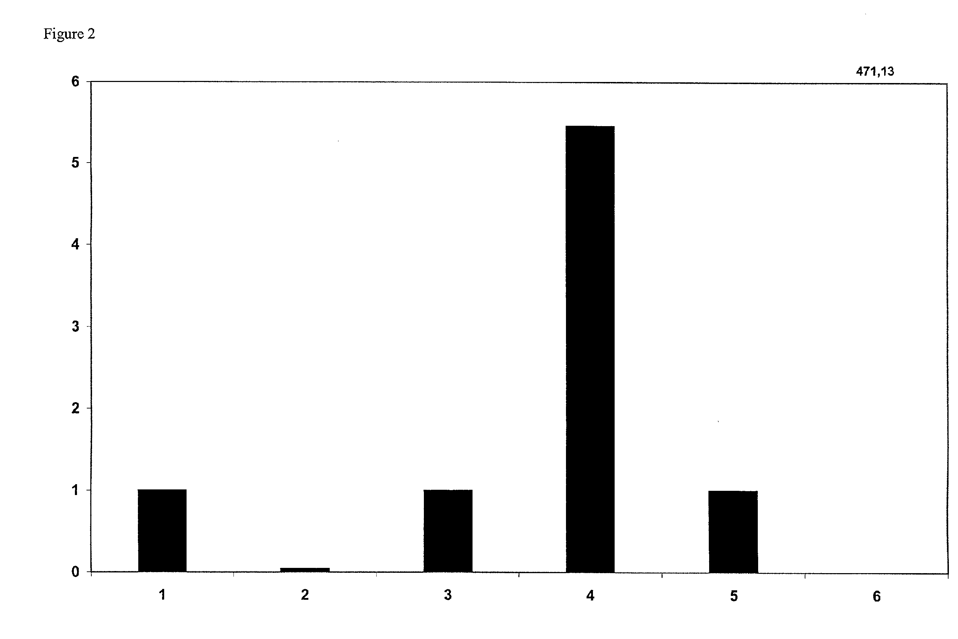

In contrast to the increased expression of P2Y₁₁R transcript during erythroid differentiation the RNA level of P2Y₁₁R decreased during the differentiation progress from early progenitor cells to granulocytes (CD15⁺ cells) in the bone marrow compartment (Figure 2, column 1 BM CD34⁺cells, column 2 BM CD15⁺ cells). The analysis of neutrophile marker gene such as G-CSF-R (figure 2, columns 3, 4) and CD11b (Figure 2, columns 5, 6) which are upregulated during the differentiation demonstrate the neutrophile character of BM CD15⁺ cells (Figure 2, BM CD34+ cells, G-CSF R column 3, CD11b column 5, BM CD15+ cells G-CSF R column 4, CD11b column 6).

The increased expression of P2Y₁₁R mRNA in erythroid cells demonstrates the important function of potential P2Y₁₁R agonists in erythropoiesis. Therefore, P2Y₁₁R agonists might be useful for the prophylaxis and / or treatment of anemia.

An object of the invention is a method of screening for therapeutic agents useful in the treatment and/or prophylaxis of anemia in a mammal comprising the steps of
i) contacting a test compound with a P2Y₁₁R polypeptide,
ii) detect binding of said test compound to said P2Y₁₁R polypeptide,
wherein those compounds are selected as potential therapeutic agents which bind to the P2Y₁₁R polypeptide.

Another object of the invention is a method of screening for therapeutic agents useful in the treatment and/or prophylaxis of anemia in a mammal comprising the steps of
i) determining the activity of a P2Y₁₁R polypeptide at a certain concentration of a test compound,
ii) determining the activity of said polypeptide in the absence or at a different concentration of said test compound,
wherein those compounds are selected as potential therapeutic agents, for which the activity of a P2Y₁₁R polypeptide as determined in i) and ii) is significantly different.

A further object of the invention is a method of screening for therapeutic agents useful in the treatment and/or prophylaxis of anemia in a mammal comprising the steps of
i) determining the activity of a P2Y₁₁R polypeptide at a certain concentration of a test compound,
ii) determining the activity of a P2Y₁₁R polypeptide at the presence of a compound known to be a regulator of a P2Y₁₁R polypeptide,
wherein those compounds are selected as potential therapeutic agents, which show an effect on the activity of a P2Y₁₁R polypeptide as determined in i), and step ii) serves as a control.

Further objects of the invention are any of the above mentioned methods, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

Another object of the invention is the method of any of the screening methods mentioned above, wherein the step of contacting is in or at the surface of a cell.

Another object of the invention is the method of any of the screening methods mentioned above, wherein the cell is in vitro.

Another object of the invention is the method of any of the screening methods mentioned above, wherein the step of contacting is in a cell-free system.

A further object of the invention is the method of any of the screening methods mentioned above, wherein the polypeptide is coupled to a detectable label.

Another object of the invention is the method of any of the screening methods mentioned above, wherein the compound is coupled to a detectable label.

Another object of the invention is the method of any of the screening methods mentioned above, wherein the test compound displaces a ligand which is first bound to the polypeptide.

A further object of the invention is the method of any of the screening methods mentioned above, wherein the polypeptide is attached to a solid support.

Another object of the invention is the method of any of claims the screening methods mentioned above, wherein the compound is attached to a solid support.

In a further embodiment, the invention provides a method of screening for therapeutic agents useful in the treatment and/or prophylaxis of anemia in a mammal comprising the steps of
i) contacting a test compound with a P2Y₁₁R polynucleotide,
ii) detect binding of said test compound to said P2Y₁₁R polynucleotide.

Furthermore, the invention provides the above mentioned method, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

Another object of the invention is the method mentioned above, wherein the nucleic acid molecule is RNA.

Another object of the invention is the method mentioned above, wherein the contacting step is in or at the surface of a cell.

A further object of the invention is the method described above, wherein the contacting step is in a cell-free system.

Another object of the invention is the method described above, wherein polynucleotide is coupled to a detectable label.

Another object of the invention is the method described above, wherein the test compound is coupled to a detectable label.

Furthermore, the invention provides a method of diagnosing anemia in a mammal comprising the steps of
i) determining the amount of a P2Y₁₁R polynucleotide in a sample taken from said mammal,
ii) determining the amount of P2Y₁₁R polynucleotide in healthy and/or diseased mammals.

Another object of the invention is the method described above, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia or idiopathic pulmonary hemosiderosis.

A further object of the invention is a pharmaceutical composition for the treatment and/or prophylaxis of anemia in a mammal comprising a therapeutic agent which binds to a P2Y₁₁R polypeptide.

Another object of the invention is a pharmaceutical composition for the treatment and/or prophylaxis of anemia in a mammal comprising a therapeutic agent which regulates the activity of a P2Y₁₁R polypeptide.

Furthermore, the invention provides a pharmaceutical composition for the treatment and/or prophylaxis of anemia in a mammal comprising a therapeutic agent which regulates the activity of a P2Y₁₁R polypeptide, wherein said therapeutic agent is
i) a small molecule,
ii) an RNA molecule,
iii) an antisense oligonucleotide,
iv) a polypeptide,
v) an antibody, or
vi) a ribozyme.

Another object of the invention is a pharmaceutical composition for the treatment and/or prophylaxis of anemia in a mammal comprising a P2Y₁₁R polynucleotide.

Another object of the invention is a pharmaceutical composition for the treatment and/or prophylaxis of anemia in a mammal comprising a P2Y₁₁R polypeptide.

A further object of the invention are the pharmaceutical compositions described above, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

Another object of the invention is the use of regulators of a P2Y₁₁R for the preparation of a pharmaceutical composition for the treatment and/or prophylaxis of anemia in a mammal.

A further object of the invention is the use of regulators of a P2Y₁₁R for the preparation of a pharmaceutical composition for the treatment and/or prophylaxis of an anemic disorder in a mammal, wherein the disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

Another object of the invention is a method for the preparation of a pharmaceutical composition useful for the treatment and/or prophylaxis of anemia in a mammal comprising the steps of
i) identifying a regulator of P2Y₁₁R,
ii) determining whether said regulator ameliorates the symptoms of anemia in a mammal; and
iii) combining of said regulator with an acceptable pharmaceutical carrier.

A further object of the invention is a method for the preparation of a pharmaceutical composition useful for the treatment and/or prophylaxis of an anemic disorder in a mammal, wherein the disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis, comprising the steps of
i) identifying a regulator of P2Y₁₁R,
ii) determining whether said regulator ameliorates the symptoms of anemia in a mammal; and
iii) combining of said regulator with an acceptable pharmaceutical carrier.

Another object of the invention is the use of a regulator of P2Y₁₁R for the regulation of P2Y₁₁R activity in a mammal having anemia.

A further object of the invention is the use of a regulator of P2Y₁₁R for the regulation of P2Y₁₁R activity in a mammal having an anemic disease comprised in a group of diseases consisting of pure red cell aplasia, anemia of chronic renal failure, anemia of endocrine disorders, congenital dyserythropoietic anemia, iron deficiency, congenital atransferrinemia and idiopathic pulmonary hemosiderosis.

### Test Compounds

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds [Lam, (1997)]. Examples of methods for the synthesis of molecular libraries can be found in the art. Libraries of compounds may be presented in solution or on beads, bacteria, spores, plasmids or phage.

### Applications

The present invention provides for prophylactic, therapeutic and / or diagnostic methods for erythropoiesis.

The regulatory method of the invention involves contacting a cell with an agent that modulates one or more of the activities of P2Y₁₁R. An agent that modulates activity can be an agent as described herein, such as a nucleic acid or a protein, a naturally-occurring cognate ligand of the polypeptide, a peptide, a peptidomimetic, or any small molecule. In one embodiment, the agent stimulates one or more of the biological activities of P2Y₁₁R. Examples of such stimulatory agents include the active P2Y₁₁R and nucleic acid molecules encoding a portion of P2Y11R. These regulatory methods can be performed in vitro (e.g., by culturing the cell with the agent) or, alternatively, in vivo (e.g, by administering the agent to a subject).

### Pharmaceutical Compositions

The nucleic acid molecules, polypeptides, and antibodies of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

An additional embodiment of the invention relates to the administration of a pharmaceutical composition containing P2Y₁₁R in conjunction with a pharmaceutically acceptable carrier, for any of the therapeutic effects discussed above. Such pharmaceutical compositions may consist of P2Y₁₁R, antibodies to P2Y₁₁R, and mimetics, agonists, antagonists, or inhibitors of P2Y₁₁R. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilisation. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration. For pharmaceutical compositions which include an antagonist of P2Y₁₁R activity, a compound which reduces expression of P2Y₁₁R, or a compound which reduces expression or activity of a protein in the P2Y₁₁R signaling pathway or any combination thereof, the instructions for administration will specify use of the composition for hematological and cardiovascular diseases, disorders of the peripheral and central nervous system, COPD, asthma, genito-urological disorders and inflammation diseases. For pharmaceutical compositions which include an agonist of P2Y₁₁R activity, a compound which increases expression of P2Y₁₁R, or a compound which increases expression or activity of a protein in the P2Y₁₁R signaling pathway or any combination thereof, the instructions for administration will specify use of the composition for hematological and cardiovascular diseases, disorders of the peripheral and central nervous system, COPD, asthma, genito-urological disorders and inflammation diseases.

### Determination of a Therapeutically Effective Dose

The determination of a therapeutically effective dose is well within the capability of those skilled in the art. A therapeutically effective dose refers to that amount of active ingredient which increases or decreases P2Y_{I1}R activity relative to P2Y₁₁R activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., ED₅₀ (the dose therapeutically effective in 50% of the population) and LD₅₀ (the dose lethal to 50% of the population), can be determined by standard pharmaceutical procedures in cell cultures or experimental animals. The dose ratio of toxic to therapeutic effects is the therapeutic index, and it can be expressed as the ratio, LD₅₀/ED₅₀. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED₅₀ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun", and DEAE- or calcium phosphate-mediated transfection.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

### Example 1: Expression Profiling

Total cellular RNA was isolated from erythroid stem and progenitor cells from bone marrow by Phenol/Chloroform extraction method with the TRIzol® Reagents according to the manufacturer's specifications (Invitrogen GmbH, Karlsruhe, Germany). To remove contamination of genomic DNA 1µg of total RNA was treated with 1 unit DNAse I (Invitrogen GmbH, Karlsruhe, Germany) for 15 minutes at room temperature. The enzyme was inactivated by addition of 1µl 25mM EDTA and incubation at 65°C for 10 minutes.

The cDNA synthesis was performed according to a modified protocol to the manufacturer's manual *(SuperScript™* II *cDNA* Synthesis *Kit,* Invitrogen GmbH, Karlsruhe, Germany). 1 µg of total RNA was mixed with 1 µl Oligo(dT)₁₂₋₁₈ (0,5 µg/ml) and DEPC-treated water (up to a final volume of 20 µl). The RNA/ Primer mix was denatured for 10 min at 70°C and immediately chilled on ice afterwards (at least 1 minute). Then 2 µl of 10x PCR Puffer, 2 µl of MgCl₂ (25 mM), 2 µl of DTT (0,1 M) and 1 µl of dNTP Mix (10 mM ) were added to the mix and preincubated for 5 minutes at 42°C. The first strand cDNA synthesis was initiated with 1 µl of SuperScript™ II RNase H⁻ Reverse Transcriptase (200 U/µl). The reaction mixture was incubated at 42°C for 50 minutes, heated for 15 minutes at 70°C and then cooled on ice. To digest the RNA 1µl of RNase H (1-4 U/µl) was added and incubated for 20 minutes at 37°C. Finally, the volume was adjusted to 100 µl with bidest. water, yielding in a final concentration of 10 ng/µl starting RNA.

The relative expression of the P2Y₁₁ receptor in human hematopoietic stem and progenitor cells was determined by quantification of the mRNA using the Real-Time Polymerase Chain Reaction (TaqMan-PCR; Heid *et al.* 1996)

To conduct the PCR reaction 7,5 µl of a Primer/Probe mix, 2,5 µl of bidest. water and 12,5 µl TaqMan Universal Master Mix (2x) (Applied Biosystems, Weiterstadt, Germany) was added to 2,5 µl of the cDNA solution. The final concentration of each primer was 300 nM and of the probe 150 nM. The primers and probes were designed using the software programme *Primer Express™* (Applied Biosystems, Weiterstadt, Germany) and synthesized by Eurogentec (Cologne, Germany). The probe was labeled with FAM (6-Carboxy-Fluorescein succinimidyl ester) as the reporter dye at the 5' end and TAMRA (6-Carboxy-tetramethyl rhodamine) as the quencher at the 3' end. The sequences of all primers and probes are listed in Table 3.

The PCR was performed using the ABI Prism SDS 7700 (Applied Biosystems, Weiterstadt, Germany) according to the manufacturer's specifications and protocols. Thermal cycling parameters were 2 min at 50°C, followed by 10 min at 95°C, followed by 40 cycles of melting at 95°C for 15 sec and annealing/extending at 60°C for 1 min. A so-called *Threshold Cycle* (C_{T}-Value) was obtained for each cDNA sample. The C_{T}-Value equals the cycle when the fluorescence intensity of the released probe has reached the 10-fold of standard deviation above the background signal. To compensate possible fluctuactions due to the cDNA synthesis the expression of a so-called *house-keeping gene* were analyzed in each cDNA sample as well. For normalization of the P2Y₁₁R expression of β-2-microglobulin was used (Table 3). The analysis of the data were performed by the so-called *ΔΔC*_{*T*} *method* according to the manufacturer's instruction (ABI Prism SDS 7700, Applied Biosystems, Weiterstadt, Germany).

### Calculation of C_{T} Values

1. The C_{T} (threshold cycle) value is calculated as follows:
   PCR reactions were set up to quantitate the housekeeping gene β-2-microglobulin (β-2-micro) for each cDNA sample.
2. C_{T(HKG)} (threshold cycle for housekeeping gene) were calculated as described in manufacture's protocol (ABI Prism SDS 7700, Applied Biosystems, Weiterstadt, Germany).
3. C_{T(gene-of-interest)} (threshold cycle for gene of interest) were calculated as described in manufacture's protocol (ABI Prism SDS 7700, Applied Biosystems, Weiterstadt, Germany).
4. ΔC_{T} (ΔC_{T} -value equals C_{T(gene-of-interest)}-value minus C_{T(HKG)}-value).
5. ΔΔC_{T} (ΔΔC_{T}-value equals ΔC_{T}-value minus ΔC_{T(Reference)}-value) The cDNA from hematopoietic stem cells from bone marrow BM CD34⁺ was used as a reference to erythroid progenitor bone marrow cells (BM CD71⁺) and neutrophile progenitor bone marrow cells (BM CD15⁺).

### Calculation of Relative Expression

Relative Expression = 2^{-(ΔΔCT)}

### Human Tissues

Total RNA of human progenitor cells derived from cord blood (CB CD34⁺), from bone marrow (BM CD34⁺ cells), erythroid progenitor bone marrow cells (BM CD71⁺), and neutrophile progenitor bone marrow cells (BM CD15⁺) were purchased from AllCells; LLC (Berkeley, California, USA).

### Expression Profile

The results of the mRNA quantification (expression profiling) is shown in Table 1 to Table 2.

**Table 1**

| Relative Expression of P2Y₁₁R, Glycophorin A (GPA), Hemoglobin alpha1/2 (Hb1/2α) and Hemoglobin beta (Hbβ) in BM CD71⁺ cells compared to BM CD34⁺ cells. | | | | | |
|---|---|---|---|---|---|
| **Cells** | Gene of interest | Relative Expression | ΔC_{T} | C_{T(gene-of-interest)} | C_{T(HKG)} (β-2-micro) |
| BM CD34⁺ | P2Y₁₁R | 1 | 9,1 | 28,06 | 18,96 |
| BM CD71⁺ | P2Y_{I1}R | 23,51 | 4,54 | 29,95 | 25,41 |
| BM CD34⁺ | GPA | 1 | 12,72 | 31,68 | 18,96 |
| BM CD71⁺ | GPA | 76862,91 | -3,51 | 21,90 | 25,41 |
| BM CD34⁺ | Hb1/2α | 1 | 7,37 | 26,33 | 18,96 |
| BM CD71⁺ | Hb1/2α | 130166,62 | -9,62 | 15,79 | 25,41 |
| BM CD34⁺ | Hb β | 1 | 2,55 | 21,51 | 18,96 |
| BM CD71⁺ | Hb β | 13587,571 | -11,18 | 14,23 | 25,41 |

**Table 2**

| Relative Expression of granulocyte colony -stimulating factor receptor (G-CSF-R) and Cluster of Differentiation 11b (CD11b) in BM CD15⁺ cells compared to BM CD34⁺ cells. | | | | | |
|---|---|---|---|---|---|
| **Cells** | Gene of interest | Relative Expression | ΔCT | C_{T(gene-of interest)} | C_{T(HKG)} (β-2-micro) |
| BM CD34⁺ | P2Y₁₁R | 1 | 10,42 | 25,5 | 15,08 |
| BM CD15⁺ | P2Y₁₁R | 0,04 | 15,19 | 33,9 | 18,72 |
| BM CD34⁺ | G-CSF-R | 1 | 5,64 | 20,72 | 15,08 |
| BM CD15⁺ | G-CSF-R | 5,46 | 3,19 | 21,91 | 18,72 |
| BM CD34⁺ | CD11b | 1 | 13,05 | 28,27 | 15,22 |
| BM CD15⁺ | CD11b | 471,13 | 4,17 | 22,89 | 18,72 |

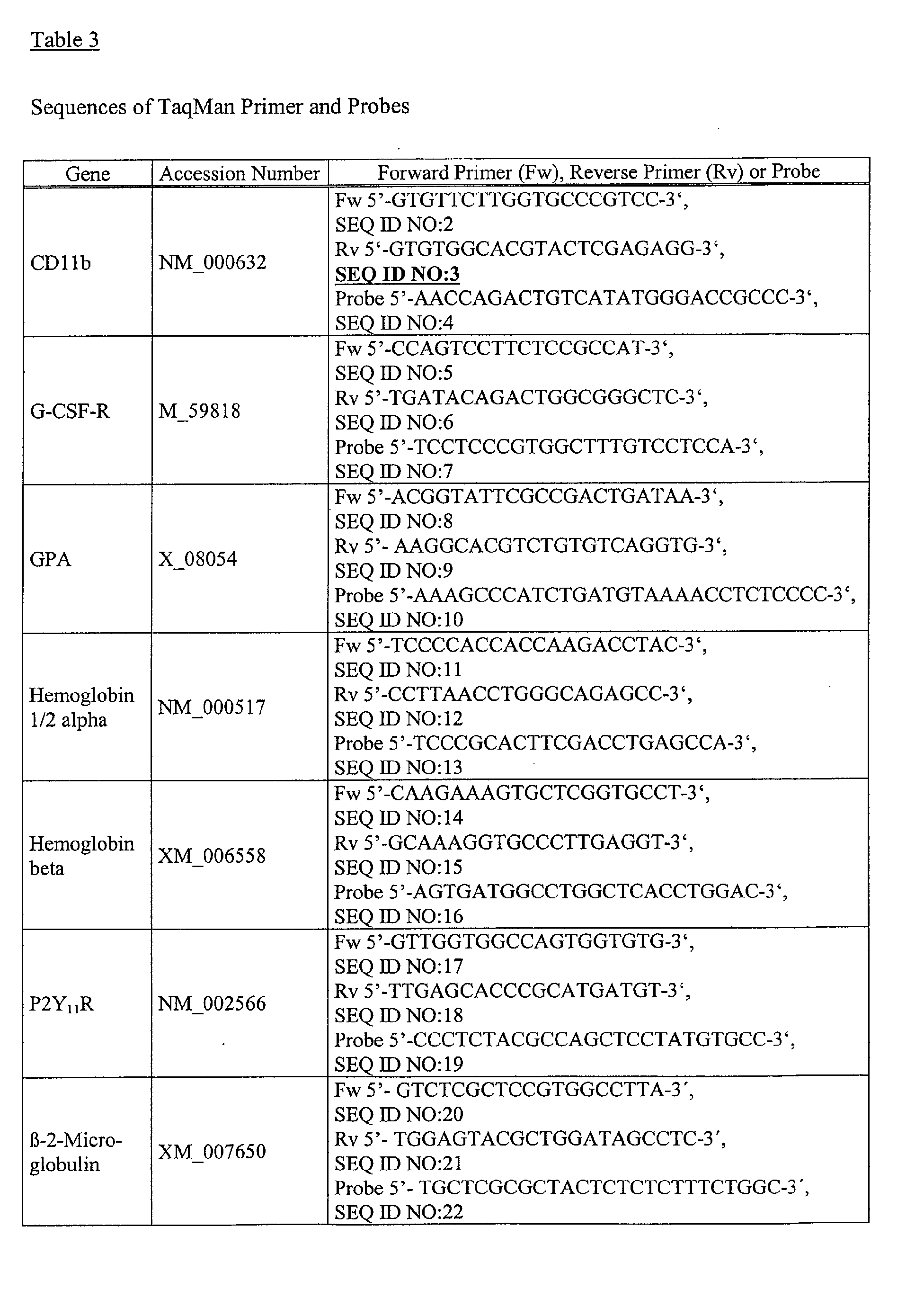

## Claims

1. A method of screening for therapeutic agents useful in the treatment and/or prophylaxis of anemia in a mammal comprising the steps of
i) contacting a test compound with a P2Y₁₁R polypeptide,
ii) detect binding of said test compound to said P2Y₁₁R polypeptide,
wherein those compounds are selected as potential therapeutic agents which bind to the P2Y₁₁R polypeptide.

2. A method of screening for therapeutic agents useful in the treatment and/or prophylaxis of anemia in a mammal comprising the steps of
i) determining the activity of a P2Y₁₁R polypeptide at a certain concentration of a test compound,
ii) determining the activity of said polypeptide in the absence or at a different concentration of said test compound,
wherein those compounds are selected as potential therapeutic agents, for which the activity of a P2Y₁₁R polypeptide as determined in i) and ii) is significantly different.

3. A method of screening for therapeutic agents useful in the treatment and/or prophylaxis of anemia in a mammal comprising the steps of
i) determining the activity of a P2Y₁₁R polypeptide at a certain concentration of a test compound,
ii) determining the activity of a P2Y₁₁R polypeptide at the presence of a compound known to be a regulator of a P2Y₁₁R polypeptide,
wherein those compounds are selected as potential therapeutic agents, which show an effect on the activity of a P2Y₁₁R polypeptide as determined in i), and step ii) serves as a control.

4. The method of any of claims 1 to 3 wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

5. A method of screening for therapeutic agents useful in the treatment and/or prophylaxis of anemia in a mammal comprising the steps of
i) contacting a test compound with a P2Y₁₁R polynucleotide,
ii) detect binding of said test compound to said P2Y₁₁R polynucleotide,
wherein those compounds are selected as potential therapeutic agents which bind to the P2Y₁₁R polynucleotide.

6. The method of claim 5, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

7. A method of diagnosing anemia in a mammal comprising the steps of
i) determining the amount of a P2Y₁₁R polynucleotide in a sample taken from said mammal,
ii) determining the amount of P2Y₁₁R polynucleotide in healthy and/or diseased mammals.

8. The method of claim 7, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

9. A pharmaceutical composition for the treatment and/or prophylaxis of anemia in a mammal comprising a therapeutic agent which binds to a P2Y₁₁R polypeptide.

10. A pharmaceutical composition for the treatment and/or prophylaxis of anemia in a mammal comprising a therapeutic agent which regulates the activity of a P2Y₁₁R polypeptide.

11. A pharmaceutical composition for the treatment and/or prophylaxis of anemia in a mammal comprising a P2Y₁₁R polynucleotide.

12. A pharmaceutical composition for the treatment and/or prophylaxis of anemia in a mammal comprising a P2Y₁₁R polypeptide.

13. The pharmaceutical compositions of claims 9 to 12, wherein the anemic disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

14. Use of regulators of a P2Y₁₁R for the preparation of a pharmaceutical composition for the treatment and/or prophylaxis of anemia in a mammal.

15. Use of regulators of a P2Y₁₁R for the preparation of a pharmaceutical composition for the treatment and/or prophylaxis of an anemic disorder in a mammal, wherein the disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis.

16. Method for the preparation of a pharmaceutical composition useful for the treatment and/or prophylaxis of anemia in a mammal comprising the steps of
i) identifying a regulator of P2Y₁₁R,
ii) determining whether said regulator ameliorates the symptoms of anemia in a mammal; and
iii) combining of said regulator with an acceptable pharmaceutical carrier.

17. Method for the preparation of a pharmaceutical composition useful for the treatment and/or prophylaxis of an anemic disorder in a mammal, wherein the disorder is selected from a group of disorders comprised of pure red cell aplasia, anemia of chronic renal failure, anemia of endocrine disorders, congenital dyserythropoietic anemia, iron deficiency, congenital atransferrinemia and idiopathic pulmonary hemosiderosis, comprising the steps of
i) identifying a regulator of P2Y₁₁R,
ii) determining whether said regulator ameliorates the symptoms of anemia in a mammal; and
iii) combining of said regulator with an acceptable pharmaceutical carrier.

**Literature**

Savi P, Beauverger P, Labouret C, Delfaud M, Salel V, Kaghad M, Herbert JM.: Role of P2Y1 purinoceptor in ADP-induced platelet activation. FEBS Lett. 1998 Feb 6;422(3):291-5.

Communi D, Gonzalez NS, Detheux M, Brezillon S, Lannoy V, Parmentier M, Boeynaems JM.: Identification of a novel human ADP receptor coupled to G(i). J Biol Chem. 2001 Nov 2;276(44):41479-85.

Conigrave AD, van der Weyden L, Holt L, Jiang L, Wilson P, Christopherson RI, Morris MB: Extracellular ATP-dependent suppression of proliferation and induction of differentiation of human HL-60 leukemia cells by distinct mechanisms. Biochem Pharmacol. 2000 Dec 1;60(11):1585-91.

Heid CA, Stevens J, Livak KJ, Williams PM: Genome Res, Real time quantitative PCR. 1996, 6 (10), 986-994.

Moore DJ, Chambers JK, Wahlin JP, Tan KB, Moore GB, Jenkins O, Emson PC, Murdock PR: Expression pattern of human P2Y receptor subtypes: a quantitative reverse transcription-polymerase chain reaction study. Biochim Biophys Acta. 2001 Oct 31;1521(1-3):107-19.

O'Reilly CM, O'Farrell AM, Ryan MP.: Purinoceptor activation of chloride transport in cystic fibrosis and CFTR-transfected pancreatic cell lines. Br J Pharmacol. 1998 Aug;124(8):1597-606.

Qi AD, Kennedy C, Harden TK, Nicholas RA.: Differential coupling of the human P2Y(11) receptor to phospholipase C and adenylyl cyclase. Br J Pharmacol. 2001 Jan;132(1):318-26.

Ralevic V, Bumstock G.: Receptors for purines and pyrimidines. Pharmacol Rev. 1998 Sep;50(3):413-92. Review.

Van der Weyden L, Adams DJ, Luttrell BM, Conigrave AD, Morris MB.: Pharmacological characterisation of the P2Y11 receptor in stably transfected haematological cell lines. Mol Cell Biochem. 2000 Oct;213(1-2):75-81.

Van der Weyden L, Conigrave AD, Morris MB.: Signal transduction and white cell maturation via extracellular ATP and the P2Y11 receptor. Immunol Cell Biol. 2000 Aug;78(4):369-74. Review.

Van der Weyden L, Rakyan V, Luttrell BM, Morris MB, Conigrave AD.: Extracellular ATP couples to CAMP generation and granulocytic differentiation in human NB4 promyelocytic leukaemia cells. Immunol Cell Biol. 2000 Oct;78(5):467-73.


SEQUENCE LISTING

<110>  Bayer AG, Bayer Health Care

<120>  P2Y11 Receptor Agonists and the Use in the Field of Erythropoiesis

<130>  Le A 36 275

<160>  22

<170>  PatentIn version 3.1

<210>  1

<211>  1116

<212>  DNA

<213>  Homo sapiens

<400>  1
atggatcgag gtgccaagtc ctgccctgcc aacttcttgg cagctgccga cgacaaactc      60

agtgggttcc agggggactt cctgtggccc atactggtgg ttgagttcct ggtggccgtg     120

gccagcaatg gcctggccct gtaccgcttc agcatccgga agcagcgccc atggcacccc     180

gccgtggtct tctctgtcca gctggcagtc agcgacctgc tctgcgctct gacgctgccc     240

ccgctggccg cctacctcta tccccccaag cactggcgct atggggaggc cgcgtgccgc     300

ctggagcgct tcctcttcac ctgcaacctg ctgggcagcg tcatcttcat cacctgcatc     360

agcctcaacc gctacctggg catcgtgcac cccttcttcg cccgaagcca cctgcgaccc     420

aagcacgcct gggccgtgag cgctgccggc tgggtcctgg ccgccctgct ggccatgccc     480

acactcagct tctcccacct gaagaggccg cagcaggggg cgggcaactg cagcgtggcc     540

aggcccgagg cctgcatcaa gtgtctgggg acagcagacc acgggctggc ggcctacaga     600

gcgtatagcc tggtgctggc ggggttgggc tgcggcctgc cgctgctgct cacgctggca 660

gcctacggcg ccctcgggcg ggccgtgcta cgcagcccag gcatgactgt ggccgagaag 720

ctgcgtgtgg cagcgttggt ggccagtggt gtggccctct acgccagctc ctatgtgccc 780

taccacatca tgcgggtgct caacgtggat gctcggcggc gctggagcac ccgctgcccg 840

agctttgcag acatagccca ggccacagca gccctggagc tggggcccta cgtgggctac 900

caggtgatgc ggggcctcat gcccctggcc ttctgtgtcc accctctact ctacatggcc 960

gcagtgccca gcctgggctg ctgctgccga cactgccccg gctacaggga cagctggaac 1020

ccagaggacg ccaagagcac tggccaagcc ctgcccctca atgccacagc cgcccctaaa 1080

ccgtcagagc cccagtcccg tgagctgagc caatga 1116

<210> 2

<211> 19

<212> DNA

<213> Homo sapiens

<400> 2
gtgttcttgg tgcccgtcc 19

<210> 3

<211> 21

<212> DNA

<213> Homo sapiens

<400> 3
gtgtggcacg tactcgagag g 21

<210> 4

<211> 26

<212> DNA

<213> Homo sapiens

<400> 4
aaccagactg tcatatggga ccgccc 26

<210> 5

<211> 18

<212> DNA

<213> Homo sapiens

<400> 5
ccagtccttc tccgccat 18

<210> 6

<211> 20

<212> DNA

<213>  Homo sapiens

<400>  6
tgatacagac tggcgggctc                                                    20

<210>  7

<211>  23

<212>  DNA

<213>  Homo sapiens

<400>  7
tcctcccgtg gctttgtcct cca                                                23

<210>  8

<211>  21

<212>  DNA

<213>  Homo sapiens

<400>  8
acggtattcg ccgactgata a                                                  21

<210>  9

<211>  21

<212>  DNA

<213>  Homo sapiens

<400>  9
aaggcacgtc tgtgtcaggt g                                                21

<210>  10

<211>  29

<212>  DNA

<213>  Homo sapiens

<400>  10
aaagcccatc tgatgtaaaa cctctcccc                                        29

<210>  11

<211>  20

<212>  DNA

<213>  Homo sapiens

<400>  11
tccccaccac caagacctac                                                  20

<210>  12

<211>  19

<212>  DNA

<213>  Homo sapiens

<400>  12
ccttaacctg ggcagagcc                                                    19

<210>  13

<211>  22

<212>  DNA

<213>  Homo sapiens

<400>  13
tcccgcactt cgacctgagc ca                                                22

<210>  14

<211>  20

<212>  DNA

<213>  Homo sapiens

<400>  14
caagaaagtg ctcggtgcct                                                   20

<210> 15

<211> 19

<212> DNA

<213> Homo sapiens

<400> 15
gcaaaggtgc ccttgaggt 19

<210> 16

<211> 24

<212> DNA

<213> Homo sapiens

<400> 16
agtgatggcc tggctcacct ggac 24

<210> 17

<211> 19

<212> DNA

<213> Homo sapiens

<400> 17
gttggtggcc agtggtgtg 19

<210> 18

<211> 19

<212> DNA

<213> Homo sapiens

<400> 18
ttgagcaccc gcatgatgt 19

<210> 19

<211> 25

<212> DNA

<213> Homo sapiens

<400> 19
ccctctacgc cagctcctat gtgcc 25

<210> 20

<211> 19

<212> DNA

<213> Homo sapiens

<400> 20
gtctcgctcc gtggcctta 19

<210> 21

<211> 21

<212> DNA

<213> Homo sapiens

<400> 21
tggagtacgc tggatagcct c 21

<210> 22

<211> 26

<212> DNA

<213> Homo sapiens

<400> 22
tgctcgcgct actctctctt tctggc 26